(19) Europäisches Patentamt
European Patent Office
Office européen des brevets

(11) Numéro de publication: **0 495 952 A1**

(12) **DEMANDE DE BREVET EUROPEEN publiée en application de l'article 158, paragraphe 3 de la CBE**

(21) Numéro de dépôt: **91914236.4**

(22) Date de dépôt: **29.07.91**

(86) Numéro de dépôt internationale : **PCT/ES91/00049**

(87) Numéro de publication internationale : **WO 92/02485 (20.02.92 92/05)**

(51) Int. Cl.[5]: **C07C 201/16**, C07C 201/12, C07C 205/57, C07C 205/58, C07C 205/59, C07C 205/60

(30) Priorité: **31.07.90 ES 9002073**

(43) Date de publication de la demande: **29.07.92 Bulletin 92/31**

(84) Etats contractants désignés: **DE FR**

(71) Demandeur: **ERCROS S.A.**
**Avda. de la Diagonal 593-595**
**ES-08014 Barcelona(ES)**

(72) Inventeur: **SAINZ DIAZ, Claro Ignacio**
**Infanta Maria Teresa 14**
**E-28016 Madrid(ES)**
Inventeur: **SANTA-OLALLA GADEA, Jesus**
**Foresta, 25**
**E-28760 Tres Cantos (Madrid)(ES)**

(74) Mandataire: **Garcia Cabrerizo, Francisco**
**OFICINA GARCIA CABRERIZO S.L. Vitruvio 23**
**E-28006 Madrid(ES)**

(54) **PROCEDE PERMETTANT D'OBTENIR DES DERIVES DE 2-NITROBENZALDEHIDES ET DES DERIVES D'ACIDES NITROBENZOIOUES.**

(57) Method for obtaining 2-nitrobenzaldehide derivatives and nitrobenzoïc acid derivatives having the formula ($I_a$) (III) respectively, wherein R is H, $C_{1-4}$ alkyl, $C_{1-4}$ alkoxy or halogen. The method is comprised of selectively oxidizing a mixture of nitrobenzaldehide derivatives (I) with an aqueous solution of an oxidizing agent (II) wherein X is halogen, M is an alkaline or alkaline-earth metal and n is 1 or 2. Through said oxidation, 3-nitrobenzaldehide derivatives essentially are oxidized. 2-nitrobenzaldehide derivatives ($I_a$) not oxidized may be removed through the use of a water-unmiscible solvent and the nitrobenzoïc acid derivatives (III) may be precipitated in the aqueous phase by pH regulation. In a preferred mode of execution, 2-nitrobenzaldehide is of pharmaceutical interest as coronary dilator and 3-nitrobenzoïc acid is of interest in the industry of dyes and in medicine as synthesis intermediary.

EP 0 495 952 A1

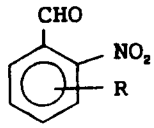

$(I_a)$

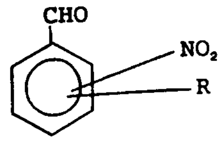

(I)

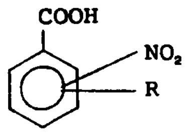

(III)

$$(XO)_n M \quad (II)$$

La présente invention se rapporte, en général, à un procédé pour séparer sélectivement des isomères de dérivés de nitrobenzaldéhydes, par oxydation sélective. En particulier, elle se rapporte à un procédé pour obtenir des dérivés purs de 2-nitrobenzaldéhyde et, alternativement, des dérivés d'acides nitrobenzoïques, par l'oxydation sélective d'un mélange de dérivés de nitrobenzaldéhydes par l'emploi de sels d'acides hypohalogéneux de métaux alcalins ou alcalino-terreux comme agents oxydants. Dans une réalisation préférée de l'invention, on obtient le 2-nitrobenzaldéhyde pur ainsi que l'acide 3-nitrobenzoïque.

L'ortho-nitrobenzaldéhyde ou 2-nitrobenzaldéhyde trouve une grande application en tant qu'intermédiaire de synthèse et, en particulier, pour la préparation de dérivés de benzodiazépines et de 4-(2'-nitropéhnyl)-1,4-dihydropyridines de grand intérêt pharmaceutique pour leur utilité en tant que dilatateur coronarien et agent hypotenseur.

La majorité des procédés classiques de synthèse des aldéhydes ne donnent pas de bons résultats pour l'obtention de 2-nitrobenzaldéhyde. Le brevet allemand 2415062, au nom de Bayer AG, décrit la synthèse du 2-nitrobenzaldéhyde par réaction du 2-nitrotoluène avec un dérivé de l'acide oxalique (un ester arylique ou dialkylique) suivie de la réaction de l'acide 2-nitrophénylpyruvique formé avec un hypochlorite alcalin et ensuite de l'hydrolyse du dichlorure de 2-nitrobenzylidène formé. Ce procédé présente l'inconvénient d'être coûteux à l'échelle industrielle, de comprendre de nombreuses étapes de réaction et de purification et de poser, en même temps, de nombreux problèmes écologiques. Le brevet allemand 2415061,au nom de Bayer AG, décrit un procédé d'obtention du 2-nitrobenzaldéhyde par réaction du 2-nitrotoluène avec un diester d'un oxalate et oxydation ultérieure avec du permanganate de potassium du 2-nitrophénylpyruvate obtenu. Ce procédé est également coûteux à l'échelle industrielle.

Un autre mode d'obtention du 2-nitrobenzaldéhyde est décrit dans le brevet allemand 2708115, au nom de Veb Arzneimittel Dresden, qui consiste à hydrolyser le bromure de 2-nitrobenzyle en alcool correspondant et ensuite à l'oxyder avec de l'acide nitrique dilué. Ce procédé est très important, nécessite de longs temps de réaction (12 heures d'hydrolyse et 5 heures d'oxydation) et en conséquence de l'oxydation avec un produit nitrique, il se forme des gazeux nitreux qui obligent à adopter des moyens coûteux pour leur absorption étant donné les normes strictes concernant leur émission.

De même, à partir du bromure de 2-nitrobenzyle, on peut obtenir le 2-nitrobenzaldéhyde par réaction avec DMSO/$CO_3K_2$ (Brevet européen 3981 au nom de Bayer AG) ou par réaction avec des oxydes de trialkylamine (Brevet Etats Unis 4335052 au nom de Dynamit Nobel AG).Ces procédés posent des problèmes à l'échelle industrielle dûs,dans un cas,à la récupération de grands volumes de DMSO et, dans l'autre cas, au maniement des oxydes d'amine et ces deux procédés sont coûteux.

D'autre part, on sait bien que le 2-nitrobenzaldéhyde se forme lors de la nitruration du benzaldéhyde conjointement avec le 3-nitrobenzaldéhyde (W. Davey et autres ; J Chem. Soc. (Londres) (1950) 208, J.W. B. Baker et autres, J. Chem. Soc. (Londres) (1931) 314, Icke et autres ; Org. Synth. coll. Vol. III page 644). Néanmoins, jusqu'à maintenant, il a été difficile et coûteux, à l'échelle industrielle, de séparer de ce mélange de nitruration, le 2-nitrobenzaldéhyde libre de ses isomères, car il n'est présent qu'en une quantité de 10-30%. Une distillation du mélange des nitrobenzaldéhydes est prohibitive pour des raisons de sécurité technique, étant donné la décomposition violente des produits. La séparation par cristallisation fractionnée n'est pas complète (O.L. Brady et autres ; J. Chem. Soc. (Londres) (1923) 484).

Un mode de séparation de ces isomères, décrit dans le brevet allemand 3212069, au nom de Bayer AG, consiste à dériver ces nitrobenzaldéhydes en forme de diméthyl et diéthylacétals avec ensuite distillation par rectification sous vide poussé et hydrolyse postérieure des dialkylacétals. Ce procédé présente une série d'inconvénients. Plus concrètement, il faut de longs temps de réaction (5 jours pour obtenir 62 g du mélange brut de diméthylacétals) ; les réactifs utilisés (méthylate de sodium, éthylate de sodium), ne sont pas faciles à manipuler à échelle industrielle et ils sont relativement peu économiques et le nombre d'opérations qu'il faut effectuer (addition d'alcool, addition d'acide, addition d'alcoolate alcalin) allonge le procédé à l'échelle industrielle. De même, ces réactifs ne sont pas récupérables, ce qui pose un problème d'effluents. D'autre part, la rectification s'effectue à peu près à 7-8 mmHg, vide très coûteux à obtenir à échelle industrielle.

C'est pourquoi la présente invention a pour objet un procédé d'obtention de dérivés de 2-nitrobenzaldéhyde qui soient purs et permettant de supprimer les inconvénients précédemment signalés, par l'oxydation sélective des isomères contenus dans un mélange de dérivés de nitrobenzaldéhydes et la séparation des dérivés de 2-nitrobenzaldéhyde purs non oxydés.

Un objet additionnel de la présente invention est constitué par un procédé pour l'obtention des dérivés des acides nitrobenzoïques obtenus par l'oxydation sélective d'un mélange de dérivés de nitrobenzaldéhydes.

L'invention se rapporte à un procédé pour obtenir des dérivés de 2-nitrobenzaldéhyde de grande pureté et, alternativement, des acides nitrobenzoïques par l'oxydation sélective d'un mélange de dérivés de

nitrobenzaldéhydes, en utilisant un sel d'acide hypohalogéneux d'un métal alcalin ou alcalino-terreux comme agent oxydant. Par ce procédé d'oxydation sélective, on oxyde principalement l'isomère correspondant au dérivé du 3-nitrobenzaldéhyde, ce qui donne lieu à des dérivés de l'acide 3-nitrobenzoïque alors que le dérivé du 2-nitrobenzaldéhyde non oxydé peut être facilement séparé. De plus, les dérivés des acides nitrobenzoïques obtenus peuvent être enlevés d'une manière facile. Cela représente un avantage considérable par rapport aux autres méthodes connues de séparation des isomères des nitrobenzaldéhydes par le fait que l'on évite d'effectuer des rectifications sous vide avec le risque conséquent d'explosions qui en découle et de même, on évite l'emploi de distillations sous vide poussé et la formation de dérivés pour séparer les isomères. Cependant, le procédé de l'invention peut être considéré comme un procédé d'obtention des dérivés de 2-nitrobenzaldéhyde de haute pureté, très économique et techniquement plus fiable que tous les procédés mentionnés antérieurement. Additionnellement, comme les produits oxydés (acides nitrobenzoïques) peuvent être facilement récupérés à une pureté élevée, le procédé global atteint un rendement élevé.

L'invention propose, d'une part, un procédé d'obtention de dérivés de 2-nitrobenzaldéhyde qui sont purs par l'oxydation sélective des isomères des dérivés des nitrobenzaldéhydes contenus dans un mélange de nitrobenzaldéhydes. De plus, on propose un procédé d'obtention de dérivés d'acides nitrobenzoïques par l'oxydation sélective de dérivés de nitrobenzaldéhydes présents dans un mélange de ceux-ci. Le mélange des dérivés de nitrobenzaldéhydes, auquel nous référerons ci-après, en tant que "mélange de nitrobenzaldéhydes" contient des composés de la formule générale (I)

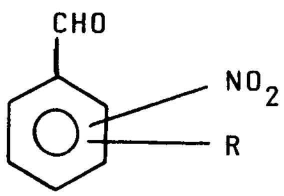

(I)

où le groupe nitro peut être en toute position 2, 3 ou 4 du noyau benzénique ; et R peut être H, un groupe alkyle de 1 à 4 atomes de carbone, un groupe alcoxy de 1 à 4 atomes de carbone ou bien un atome d'halogène, et il peut être en toute position du noyau benzénique non occupée par le groupe nitro.

L'expression groupe alkyle de 1 à 4 atomes de carbone, dans le sens utilisé dans cette description, indique des résidus d'hydrocarbures à chaîne linéaire ou ramifiée contenant de 1 à 4 atomes de carbone.

L'expression groupe alcoxy de 1 à 4 atomes de carbone indique des résidus qui dérivent d'un alcool à chaîne linéaire ou ramifiée de 1 à 4 atomes de carbone, préférentiellement méthoxy ou éthoxy.

Le mélange de nitrobenzaldéhydes (I) peut être de toute origine ou peut être obtenu par des méthodes connues de nitruration des benzaldéhydes. En particulier, un mélange de nitrobenzaldéhydes de ceux utilisés pour la réalisation de la présente invention contient des dérivés de nitrobenzaldéhydes de la formule (I) précédemment indiquée, avec 10% en poids à 48% en poids du dérivé de 2-nitrobenzaldéhyde, 90% en poids à 50% en poids du dérivé de 3-nitrobenzaldéhyde et, occasionnellement, 0% à 2% en poids du dérivé de 4-nitrobenzaldéhyde.

Les agents oxydants adéquats pour l'oxydation sélective et majoritaire des dérivés de 3- et 4-nitrobenzaldéhyde contenus dans ce mélange de nitrobenzaldéhydes, sont des sels d'acides hypohalogéneux de métaux alcalins ou alcalino-terreux de formule générale (II)

$(XO)_nM$ (II)

où

X est un atome d'halogène ;
M est un métal alcalin ou alcalino-terreux ; et
n est 1 ou 2.

Parmi ces agents oxydants de formule (II), on préfère particulièrement, pour réaliser le procédé de la présente invention, l'hypochlorite de sodium et l'hypochlorite de calcium. Le pouvoir oxydant de ces hypochlorites est compris entre environ 5% et 65% en chlore actif.

Aussi bien l'hypochlorite de sodium que l'hypochlorite de calcium sont de forts oxydants capables d'oxyder des benzaldéhydes, des arylcétones, des alkylbenzènes et des alcools aryliques et de les convertir en acides benzoïques. De même, ils sont également capables d'oxyder des aldéhydes et de les convertir en acides carboxyliques. Cependant, il s'est révélé de manière surprenante que ces hypochlorites,

dans des conditions adéquates, sont capables d'oxyder sélectivement et majoritairement les dérivés de 4- et 3-nitrobenzaldéhyde qui sont contenus dans un mélange de dérivés de nitrobenzaldéhydes (I), tandis que les dérivés de 2-nitrobenzaldéhyde sont oxydés en petites proportions, c'est pourquoi on peut les séparer du mélange de nitrobenzaldéhydes. Ce fait constitue l'un des aspects essentiels de la présente invention.

L'un des objets de l'invention est un procédé d'obtention de dérivés de 2-nitrobenzaldéhyde qui sont purs ($I_a$)

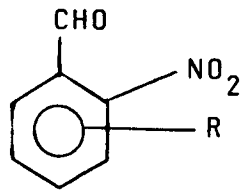

($I_a$)

où R a les significations mentionnées pour la formule (I) antérieurement indiqué.
qui sont contenus dans un mélange de nitrobenzaldéhydes de formule (I) précédemment indiquée, au moyen d'un procédé qui comprend les étapes suivantes :

a) faire réagir un mélange de nitrobenzaldéhydes de formule générale (I) avec un agent oxydant de formule (II) précédemment indiquée ; et

b) retirer les dérivés de 2-nitrobenzaldéhydes non oxydés ($I_a$) par extraction au moyen d'un solvant adéquat.

Comme antérieurement mentionné, le mélange de nitrobenzaldéhydes de formule (I) peut être obtenu par des méthodes conventionnelles et peut contenir 10% en poids jusqu'à 48% en poids du dérivé de 2-nitrobenzaldéhyde ($I_a$) et 90% en poids jusqu'à 50% en poids du dérivé de 3-nitrobenzaldéhyde ($I_b$)

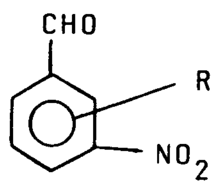

($I_b$)

où R a les significations mentionnées pour la formule (I) antérieurement indiquée,
et à l'occasion, de 0% à 2% en poids du dérivé de 4-nitrobenzaldéhyde.

Si le mélange des nitrobenzaldéhydes de formule (I) est en phase solide, il faut la dissoudre avant d'effectuer la réaction d'oxydation. Pour dissoudre ce mélange, on peut utiliser tous les solvants organiques capables de dissoudre les dérivés de nitrobenzaldéhydes de formule (I) qui sont contenus dans le mélange. Ces solvants peuvent être aussi bien miscibles qu'immiscibles avec l'eau. Le choix d'un type ou l'autre de solvant permet la réalisation de légères variantes du procédé général de l'invention que l'on mentionnera ultérieurement. A titre d'exemple, on peut citer comme solvants adéquats du mélange des nitrobenzaldéhydes, l'acétonitrile, le dichlorométhane, le benzène, le toluène ou le xylène. En particulier, on a observé que l'acétonitrile (miscible avec l'eau) et le dichloroéthane (immiscible) sont des solvants qui sont tout à fait appropriés pour réaliser le procédé de l'invention, pour citer 2 exemples représentatifs de chaque groupe de solvants.

L'agent oxydant de formule (II) se présente sous la forme d'une solution aqueuse. Cette solution aqueuse est constituée d'une dissolution de l'agent oxydant (II) dans l'eau et d'un acide afin de diminuer le pH de la solution de l'agent oxydant jusqu'à des valeurs de pH qui sont comprises entre 4 et 11, de préférence 5 et 9, parce que c'est dans cet intervalle de pH que l'on a observé que l'oxydation sélective des dérivés de 3-nitrobenzaldéhyde ($I_b$) se produisait au mieux.

L'acide présent dans la solution aqueuse de l'agent oxydant (II) peut être un acide organique miscible avec l'eau ou un acide inorganique. Alternativement, on peut également utiliser des résines acides d'échange d'ions en suspension. La quantité de cet acide présent dans la solution aqueuse de l'agent oxydant (II) dépend de la valeur du pH à laquelle on souhaite réaliser la réaction d'oxydation.

La réaction d'oxydation (étape a) est effectuée en mettant le mélange de nitrobenzaldéhydes de formule (I) précédemment dissous, en contact avec la solution aqueuse de l'agent oxydant (II). En général, il est pratique d'effectuer l'oxydation à de basses températures, généralement comprises entre -20 ° C et

+25 ° C, à un pH compris entre 4 et 11, de préférence entre 5 et 9, et avec agitation.

La relation molaire entre les dérivés de nitrobenzaldéhydes (I) contenus au total dans le mélange et l'agent oxydant (II) peut être comprise entre 1:1-3 respectivement (1 mole des nitrobenzaldéhydes pour 1 à 3 moles de l'agent oxydant), étant de préférence comprise entre 1:1,5-2.

Pendant la réaction d'oxydation, on mesure périodiquement la teneur en dérivé de 3-nitrobenzaldéhyde ($I_b$) présent dans le milieu réactionnel par chromatographie en couche mince (TLC) et par chromatographie liquide de haute résolution (HPLC). Lorsque l'on ne détecte pas de dérivé du 3-nitrobenzaldéhyde, on considère que la réaction d'oxydation est terminée. En opérant selon les conditions précédemment indiquées, l'oxydation peut durer entre 10 heures et 60 heures selon la température de travail.

Une fois finie la réaction d'oxydation, tout le dérivé de 3-nitrobenzaldéhyde ($I_b$) présent initialement dans le mélange des nitrobenzaldéhydes,ainsi qu'une petite partie du dérivé de 2-nitrobenzaldéhyde ($I_a$) et tout le dérivé possible de 4-nitrobenzaldéhyde qui peut exister dans le mélange initial se sont convertis en dérivés correspondants des acides 3-, 2- et 4-nitrobenzoïques (III)

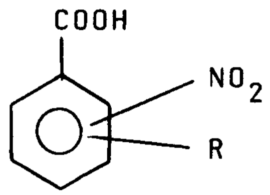

(III)

où R a les significations indiquées pour la formule (I) précédemment démontrée et le groupe nitro peut être en toutes positions du noyau benzénique,
tandis qu'une grande partie du dérivé de 2-nitrobenzaldéhyde ($I_a$) originellement présent dans le mélange de nitrobenzaldéhydes, reste telle quelle, c'est-à-dire sans s'oxyder, et on procède alors à son extraction du milieu réactionnel. L'extraction du dérivé de 2-nitrobenzaldéhyde ($I_a$) non oxydé, du milieu réactionnel, est effectuée par extraction au moyen d'un solvant adéquat. La stratégie d'extraction dépend du type de solvant utilisé pour dissoudre le mélange des nitrobenzaldéhydes. Comme on l'a précédemment mentionné ce solvant peut être miscible ou immiscible avec l'eau.

Lorsque le solvant est miscible avec l'eau (par exemple l'acétonitrile), la réaction d'oxydation se produit très rapidement et l'extraction du dérivé de 2-nitrobenzaldéhyde ($I_a$) non oxydé peut être effectuée par la basification du milieu réactionnel et l'addition d'un solvant organique immiscible avec l'eau afin de retirer le dérivé ($I_a$) non oxydé, lequel reste dans la phase organique. Cette phase organique est séchee, est filtrée et est concentrée en succession, pour ainsi obtenir le dérivé de 2-nitrobenzaldéhyde pur ($I_a$). Le solvant adéquat pour effectuer l'extraction de ces composés ($I_a$) peut être tout solvant immisible avec l'eau et dans lequel le dérivé de 2-nitrobenzaldéhyde sera soluble, tel que le dichlorométhane, le benzène, le toluène ou le xylène. Les bases adéquates pour la basification du milieu réactionnel peuvent être celles qui permettent la formation de sels des acides nitrobenzoïques formés comme la soude diluée, le bicarbonate de soude, l'hydroxyde d'ammonium et analogues, pour en citer quelques exemples.

Quand le solvant du mélange de nitrobenzaldéhydes (I) est un solvant immiscible avec l'eau (comme le dichlorométhane), la réaction d'oxydation est plus lente ainsi que plus sélective. Afin d'augmenter la vitesse de la réaction,on peut ajouter, au milieu réactionnel, un catalyseur de transfert de phase parmi ceux habituellement utilisés tels que les sels d'ammonium quaternaire. Quand la réaction d'oxydation est terminée, il y a dans la phase organique le dérivé de 2-nitrobenzaldéhyde ($I_a$) qui n'est pas oxydé joint aux acides nitrobenzoïques (III) formés. Pour extraire le composé ($I_a$) dans une première étape, on basifie le milieu par l'addition d'une base parmi celles précédemment citées, afin de former les sels des acides nitrobenzoïques (III) formés pour que ceux-ci passent à la phase aqueuse afin qu'il ne reste, dans la phase organique, que le dérivé de 2-nitrobenzaldéhyde qui,postérieurement, est séche, est filtré et est concentré afin d'obtenir le composé ($I_a$) pur. A la phase aqueuse séparée,on peut ajouter de nouveau un solvant immiscible avec l'eau afin d'extraire le dérivé possible de 2-nitrobenzaldéhyde ($I_a$) qui pourrait être resté dans cette phase aqueuse. Pour des raisons pratiques, il est commode que ce solvant d'extraction soit le même que celui utilisé pour dissoudre le mélange de nitrobenzaldéhydes (I).

Dans un objet additionnel, la présente invention propose un procédé d'obtention de dérivés d'acides nitrobenzoïques de formule générale (III)

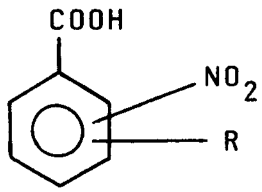

(III)

où le groupe nitro peut être en toute position 2, 3 ou 4 du noyau benzénique, et
R est H, un groupe alkyle ou alcoxy de 1 à 4 atomes de carbone ou bien un atome d'halogène et peut être en toute position du noyau benzénique qui n'est pas occupée par le groupe nitro.

Par groupe alkyle et alcoxy de 2 à 4 atomes de carbone, on indique ceux mentionnés par rapport à la formule (I).

Dans une première alternative, ces acides nitrobenzoïques (III) peuvent être obtenus à partir des phases aqueuses résultant de l'extraction du dérivé de 2-nitrobenzaldéhyde ($I_a$). En effectuant l'oxydation sélective du mélange de nitrobenzaldéhydes de la formule (I) antérieurement montrée, avec la solution aqueuse de l'agent oxydant (II), précédemment défini, on produit l'oxydation totale du dérivé de 3-nitrobenzaldéhyde ($I_b$) et du dérivé de 4-nitrobenzaldéhyde dans le cas où il y en avait dans le mélange initial et d'une partie du dérivé de 2-nitrobenzaldéhyde ($I_a$) contenus dans le mélange des nitrobenzaldéhydes de formule (I) pour ainsi obtenir les acides nitrobenzoïques (III) correspondants. Plus concrètement, on obtient majoritairement le dérivé de l'acide 3-nitrobenzoïque ($III_a$)

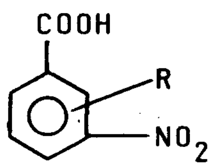

($III_a$)

où R a les significations mentionnées par rapport à la formule (III)
et en une moindre proportion, le dérivé de l'acide 2-nitrobenzoïque ($III_b$)

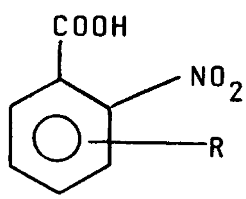

($III_b$)

où R a les significations mentionnées par rapport à la formule (III).

Ces acides nitrobenzoïques (III), une fois extrait le dérivé de 2-nitribenzaldéhyde dans la phase organique, se trouvent dans la phase aqueuse sous la forme des sels formés avec le cation de la base utilisée pour la basification du milieu réactionnel. Les sels de ces acides nitrobenzoïques (III) sont solubles dans l'eau tandis que les acides (III) ne le sont pas. Par conséquent, en acidifiant le milieu réactionnel par un acide,on peut précipiter sélectivement les acides ($III_a$) et ($III_b$) en vertu des différentes valeurs de pKa.

Comme acides adéquats pour abaisser le pH et provoquer la précipitation de ces acides nitrobenzoïques, on peut utiliser tout acide fort, tel que chlorhydrique, sulfurique, nitrique, phosphorique et analogues.

En contrôlant le pH à une valeur de l'ordre de la valeur de pKa du dérivé de l'acide 3-nitrobenzoïque ($III_a$),celui-ci peut être séparé par précipitation et il reste,dans la solution,le dérivé de l'acide 2-nitrobenzoïque ($III_b$) qui précipite une fois que la totalité de l'acide ($III_a$) a été retirée du milieu réactionnel. Ainsi, en abaissant à un pH compris entre 3,5 et 4,5, le dérivé de l'acide 3-nitrobenzoïque ($III_a$) précipite. Une fois que l'on a prouvé que la totalité de l'acide ($III_a$) était retirée du milieu réactionnel, on ajoute de nouveau de l'acide jusqu'à un pH d'environ 1 (pH environ 0,8 - 1,2) pour provoquer la précipitation du dérivé de l'acide 2-nitrobenzoïque ($III_b$).

Ce procédé d'obtention des acides nitrobenzoïques ($III_a$) et ($III_b$) peut être réalisé aussi bien de façon

discontinue que continue. En mode discontinu, on ajoute l'acide à la phase aqueuse résultant de l'extraction du dérivé de 2-nitrobenzaldéhyde ($I_a$) jusqu'à atteindre une valeur du pH comprise entre 3,5 et 4,5 pour ainsi provoquer la précipitation du dérivé de l'acide 3-nitrobenzoïque ($III_a$) que l'on retire du milieu par filtration. Les eaux mères sont de nouveau acidifiées le nombre de fois nécessaire pour que la valeur du pH du milieu réactionnel soit maintenue constante aux environs de 3,5. A ce moment, on considère que tout le dérivé de l'acide 3-nitrobenzoïque s'est retiré du milieu réactionnel. En opérant dans ces conditions, il est possible d'obtenir un rendement lors de la récupération du dérivé de l'acide 3-nitrobenzoïque ($III_a$) présent dans la phase aqueuse de départ supérieur à 94% et un acide ($III_a$) d'une pureté supérieure 97%. En mode continu, le procédé peut être effectué facilement par mise en recirculation de la solution aqueuse des acides nitrobenzoïques (III) en contrôlant le pH et en filtrant. Pour cela, la solution acidulée de sortie du récipient de réaction est remise en circulation et on la fait passer par un filtre où est retenu le dérivé de l'acide 3-nitrobenzoïque ($III_a$) ainsi que les liquides mères, contenant le dérivé de l'acide 2-nitrobenzoïque ($III_b$) et un peu du dérivé ($III_a$) qui n'a pas précipité, pour une nouvelle introduction dans le récipient de réaction.

Alternativement, dans un mode préféré de réalisation, l'invention propose un procédé d'obtention de dérivés de l'acide 3-nitrobenzoïque de formule générale ($III_a$), montrée précédemment, par l'oxydation sélective d'un mélange de nitrobenzaldéhydes de formule (I) antérieurement indiquée, avec une solution aqueuse d'un agent oxydant (II) précédemment défini. Le mélange des nitrobenzaldéhydes (I) peut contenir la même composition en poids que celle précédemment indiquée.

Ce procédé comprend les étapes de :

a) faire réagir le mélange de nitrobenzaldéhydes de formule générale (I) avec l'agent oxydant de formule (II) et

b) récupérer le dérivé de l'acide 3-nitrobenzoïque ($III_a$) formé.

La réaction d'oxydation de ce mélange de nitrobenzaldéhydes (I) se passe de la même manière qu'on l'a précédemment décrit, c'est-à-dire que l'on met en contact le mélange dissous des dérivés de nitrobenzaldéhyde (I) avec une solution aqueuse de l'agent oxydant (II), à un pH compris entre 4 et 11, de préférence à un pH compris entre 5 et 9 et à une température comprise entre -20 ° C et +25 ° C et sous agitation mais, dans ce cas, on ne traite pas le dérivé de 3-nitrobenzaldéhyde ($I_b$) présent initialement dans le mélange de nitrobenzaldéhydes (I). En effet, afin d'obtenir le dérivé de l'acide 3-nitrobenzoïque ($III_a$) avec un grand rendement et une grande pureté et d'éviter la réalisation d'étapes additionnelles de purification, il est pratique que la réaction d'oxydation soit suivie de la conversion du dérivé de 3-nitrobenzaldéhyde ($I_b$) et d'arrêter la réaction lorsque la relation entre la conversion du dérivé de 3-nitrobenzaldéhyde ($I_b$) et du dérivé de 2-nitrobenzaldéhyde ($I_a$) est optimale et permet une séparation quantitative de l'acide 3-nitrobenzoïque ($III_a$) formé avec une haute pureté. Nous avons observé qu'en arrêtant la réaction lorsque la conversion du dérivé de 3-nitrobenzaldéhyde ($I_b$) est de l'ordre de 90%, la conversion du dérivé de 2-nitrobenzaldéhyde ($I_a$) est de 25% et, de cette manière, on peut facilement obtenir, par le procédé décrit précédemment de séparation du dérivé de l'acide 3-nitrobenzoïque ($III_a$) par précipitation avec contrôle du pH, un acide ($III_a$) d'une pureté supérieure à 97% et avec un rendement supérieur à 94%.

Les acides 3-nitrobenzoïques ($III_a$) ainsi obtenus peuvent être utilisés en tant que produits de départ pour l'obtention d'autres dérivés tels que les acides 3,5-dinitrobenzoïques de grand intérêt dans l'industrie des colorants et des médicaments.

L'invention sera plus particulièrement décrite par les exemples qui suivent mais, cependant, il faut comprendre que l'invention n'est pas limitée à ces exemples et que l'on peut y apporter diverses modifications sans s'écarter du cadre et de l'esprit de la présente invention.

EXEMPLES

Dans les exemples qui suivent, la réaction d'oxydation est effectuée en déterminant la quantité du 3-nitrobenzaldéhyde présent dans le milieu réactionnel par TLC et HPLC quand sa teneur est de l'ordre de 1%. Le 2-nitrobenzaldéhyde obtenu pur est déterminé et quantifié au moyen de HPLC et RMN.

Exemple 1

On dissout 5 g d'un mélange de nitrobenzaldéhydes provenant de la nitruration du benzaldéhyde, avec 77% de 3-nitrobenzaldéhyde et 22% de 2-nitrobenzaldéhyde dans 40 ml d'acétonitrile et on ajoute lentement un mélange de 8 g d'hypochlorite de calcium (65% de chlore actif), de 10 ml d'acide acétique et de 100 ml d'eau. Au bout de 48 heures à -10 ° C, on ajoute 50 ml de dichlorométhane et une solution aqueuse saturée de bicarbonate de soude jusqu'à ce que la phase aqueuse soit basique. On décante et la

phase aqueuse est soumise à extraction avec 3 x 50 ml de dichlorométhane. Les phases organiques sont réunies, séchées avec du sulfate de sodium anhydre, filtrées et concentrées afin d'obtenir 0,22 g de 2-nitrobenzaldéhyde pur.

Exemple 2

Une solution de 5 g d'un mélange de nitrobenzaldéhydes, avec 77% de 3-nitrobenzaldéhyde et 22% de 2-nitrobenzaldéhyde, dans 40 ml d'acétonitrile, est lentement ajoutée à un mélange de 50 ml d'hypochlorite de sodium (11% de chlore actif) et de 10 ml d'acide acétique. Au bout de 43 heures à -15 ° C, on ajoute 50 ml de dichlorométhane et une solution aqueuse saturée de bicarbonate de soude jusqu'à ce que la phase aqueuse soit basique. On décante et la phase aqueuse est extraite avec 3 x 50 ml de dichlorométhane. Les phases organiques sont réunies, séchées avec du sulfate de sodium anhydre, filtrées et concentrées pour obtenir 0,55 g de 2-nitrobenzaldéhyde (pureté > 95%).

Exemple 3

Une solution de 10 g du mélange de nitrobenzaldéhydes, avec 77% de 3-nitrobenzaldéhyde et 22% de 2-nitrobenzaldéhyde, dans 30 ml d'acétonitrile, est ajoutée à un mélange de 10 ml d'hypochlorite de sodium (11% de chlore actif) et de 4 ml d'acide acétique. Au bout de 48 heures à -13 ° C, on ajoute 100 ml de dichlorométhane et une solution aqueuse saturée de bicarbonate de soude jusqu'à ce que la phase aqueuse soit à un pH basique. En suivant le même procédé de l'exemple antérieur, on obtient 0,70 g de 2-nitrobenzaldéhyde pur.

Exemple 4

Une solution de 10 g d'un mélange de nitrobenzaldéhydes avec une proportion de 77/22 de 3-nitro/2-nitrobenzaldéhydes, respectivement, dans 30 ml de dichlorométhane, est ajoutée à un mélange de 100 ml d'hypochlorite de sodium (11% de chlore actif) et de 4 ml d'acide acétique. Au bout de 100 heures à 25 ° C, dans un réacteur et sous forte agitation, on basifie le mélange de réaction et en suivant le procédé d'extraction de l'exemple 1,on obtient 0,4 g de 2-nitrobenzaldéhyde pur.

Exemple 5

Une solution de 10 g d'un mélange de nitrobenzaldéhydes avec une proportion de 77% et 22% de 3- et 2-nitrobenzaldéhyde,respectivement, dans 30 ml d'acétonitrile, est ajoutée à un mélange de 100 ml d'hypochlorite de sodium (11% de chlore actif) et de 4 ml d'acide acétique. Au bout de 15 heures à -15 ° C, on basifie le mélange de réaction au moyen de bicarbonate de soude et on ajoute 100 ml de dichlorométhane. La phase organique est extraite avec 2 x 50 ml d'une solution aqueuse de bicarbonate de soude. On réunit les phases aqueuses, on les lave avec 50 ml de dichlorométhane et on acidifie à pH 3,5-4, pour obtenir 7,2 g d'un précipité blanc-jaunâtre identifié comme étant l'acide 3-nitrobenzoïque pur (pureté supérieure à 97%).

**Revendications**

**1.** Procédé pour l'obtention de dérivés de 2-nitrobenzaldéhydes de formule ($I_a$)

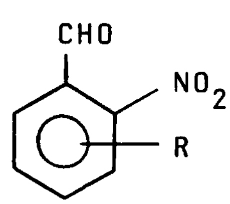

($I_a$)

où R peut être H, un groupe alkyle de 1 à 4 atomes de carbone, un groupe alcoxy de 1 à 4 atomes de carbone ou un atome d'halogène et peut être en toute position du noyau benzénique non occupée par le groupe nitro,
par l'oxydation sélective d'un mélange de nitrobenzaldéhydes de formule (I)

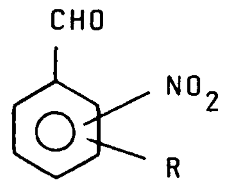

(I)

où le groupe nitro peut se trouver en l'une quelconque des positions 2, 3 ou 4 du noyau benzénique ;

et R peut être H, un groupe alkyle de 1 à 4 atomes de carbone, un groupe alcoxy de 1 à 4 atomes de carbone ou un atome d'halogène et peut être en toute position du noyau benzénique non occupée par le groupe nitro,

ledit procédé étant caractérisé en ce qu'il consiste à

a) faire réagir un mélange dissous des dérivés de nitrobenzaldéhydes de la formule (I) avec un agent oxydant de formule (II)

$(XO)_nM$ (II)

où

X est un halogène ;

M est un métal alcalin ou alcalino-terreux ; et

n est 1 ou 2 ; et

b) séparer le dérivé de 2-nitrobenzaldéhyde pur ($I_a$) non oxydé.

**2.** Procédé selon la revendication 1, caractérisé en ce que le mélange des dérivés des nitrobenzaldéhydes contient 10% en poids à 48% en poids du dérivé de 2-nitrobenzaldéhyde, 90% en poids à 50% en poids du dérivé de 3-nitrobenzaldéhyde, et facultativement 0% en poids à 2% en poids du dérivé de 4-nitrobenzaldéhyde.

**3.** Procédé selon la revendication 1, caractérisé en ce que le solvant du mélange des dérivés de nitrobenzaldéhydes (I) peut être un solvant organique miscible ou immiscible avec l'eau.

**4.** Procédé selon la revendication 3, caractérisé en ce que, lorsque le solvant est immiscible avec l'eau, on ajoute un catalyseur de transfert de phase au milieu réactionnel.

**5.** Procédé selon la revendication 1, caractérisé en ce que l'agent oxydant est choisi parmi l'hypochlorite de sodium ou l'hypochlorite de calcium.

**6.** Procédé selon la revendication 1, caractérisé en ce que l'agent oxydant est sous forme de solution aqueuse en mélange avec un acide inorganique ou avec un acide organique miscible avec l'eau.

**7.** Procédé selon la revendication 1, caractérisé en ce que la réaction d'oxydation est effectuée à un pH compris entre 4 et 11, de préférence entre 5 et 9.

**8.** Procédé selon la revendication 1, caractérisé en ce que l'oxydation est effectuée à une température comprise entre -20 ° C et 25 ° C.

**9.** Procédé selon la revendication 1, caractérisé en ce que le dérivé de 2-nitrobenzaldéhyde non oxydé est séparé du milieu réactionnel par extraction au moyen d'un solvant organique immiscible avec l'eau.

**10.** Procédé selon la revendication 9, caractérisé en ce que le solvant est choisi parmi le benzène, le toluène, le xylène, le dichlorométhane, le dichloroéthane ou le chloroforme.

**11.** Procédé d'obtention de dérivés d'acides nitrobenzoïques de formule générale (III)

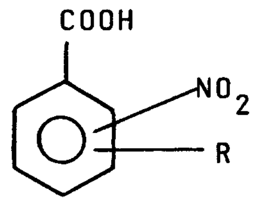

(III)

où le groupe nitro peut être en toute position 2, 3 ou 4 du noyau benzénique ; et
R peut être H, un groupe alkyle de 1 à 4 atomes de carbone ou un groupe alcoxy de 1 à 4 atomes de carbone ou un atome d'halogène et peut être en toute position du noyau benzénique non occupée par le groupe nitro,
par oxydation sélective, caractérisé en ce qu'il consiste à :

a) faire réagir un mélange dissous des dérivés des nitrobenzaldéhydes de formule (I)

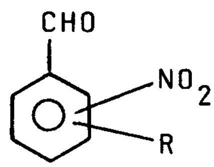

(I)

où le groupe nitro peut être en toute position 2, 3 ou 4 du noyau benzénique ; et
R a les significations précédemment indiquées ;
avec une solution aqueuse d'un agent oxydant de formule (II)

$(XO)_nM$ (II)

où
X est un halogène ;
M est un métal alcalin ou alcalino-terreux ; et
n est 1 ou 2 ;

b) ajouter un solvant immiscible avec l'eau,
c) basifier le milieu réactionnel,
d) retirer la phase obtenue ; et
e) précipiter les dérivés des acides nitrobenzoïques (III) présents dans la phase aqueuse sous la forme de sels, par acidification contrôlée.

**12.** Procédé selon la revendication 11, caractérisé en ce que le mélange des dérivés des nitrobenzaldéhydes (I) contient 10% en poids à 48% en poids du dérivé de 2-nitrobenzaldéhyde, 90% en poids à 50% en poids du dérivé de 3-nitrobenzaldéhyde et 0% en poids à 2% en poids du dérivé de 4-nitrobenzaldéhyde.

**13.** Procédé selon la revendication 11, caractérisé en ce que le solvent du mélange des dérivés des nitrobenzaldéhydes (I) peut être miscible ou immiscible avec l'eau.

**14.** Procédé selon la revendication 11, caractérisé en ce que l'agent oxydant est choisi parmi l'hypochlorite de sodium ou l'hypochlorite de calcium.

**15.** Procédé selon la revendication 11, caractérisé en ce que l'agent oxydant est sous la forme d'une solution aqueuse mélangée à un acide inorganique ou un acide organique miscible avec l'eau.

**16.** Procédé selon la revendication 11, caractérisé en ce que le solvant immiscible avec l'eau utilisé à l'étape b) est choisi parmi le benzène, le toluène, le xylène, le dichlorométhane, le dichloroéthane ou le chloroforme.

**17.** Procédé selon la revendication 11, caractérisé en ce que, pour basifier le milieu, on ajoute des bases

inorganiques, telles que de la soude, de la potasse, de l'hydroxyde d'ammonium ou du bicarbonate de soude.

**18.** Procédé selon la revendication 11, caractérisé en ce qu'en contrôlant le pH à des valeurs comprises entre 3,5 et 4,5, le dérivé de l'acide 3-nitrobenzoïque précipite, que l'on retire par filtration.

**19.** Procédé selon la revendication 11, caractérisé en ce qu'une fois que la totalité du dérivé de l'acide 3-nitrobenzoïque a été retirée, à un pH proche de 1, le dérivé de l'acide 3-nitrobenzoïque précipite.

**20.** Procédé selon la revendication 11, caractérisé en ce que la précipitation des dérivés des acides nitrobenzoïques par contrôle du pH peut s'effectuer de façon continue ou discontinue.

**21.** Procédé pour l'obtention des dérivés de l'acide 3-nitrobenzoïque de formule ($III_a$)

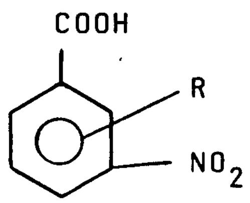

($III_a$)

où R a la signification indiquée par rapport à la formule (III) précédemment montrée caractérisé en ce qu'il consiste à :

a) faire réagir un mélange de dérivés de nitrobenzaldéhydes de formule (I) précédemment montrée avec une solution aqueuse d'un agent oxydant de formule (II)

$(XO)_nM$ (II)

où

- X est un halogène ;
- M est un métal alcalin ou alcalino-terreux ; et
- n est 1 ou 2 ;

b) arrêter la réaction d'oxydation des dérivés des nitrobenzaldéhydes (I) quand la relation entre la conversion du dérivé de 3-nitrobenzaldéhyde et les conversions des autres isomères présents dans le mélange initial est la mieux adéquate.

c) récupérer le dérivé de l'acide 3-nitrobenzoïque ($III_a$) formé.

**22.** Procédé selon la revendication 21, caractérisé en ce que la réaction d'oxydation est arrêtée quand la conversion du dérivé de 3-nitrobenzaldéhyde est de l'ordre de 90%.

**23.** Procédé selon la revendication 21, caractérisé en ce que pour arrêter la réaction d'oxydation, on ajoute un solvant immiscible avec l'eau et on basifie le milieu réactionnel.

**24.** Procédé selon la revendication 21, caractérisé en ce que le dérivé de l'acide 3-nitrobenzoïque ($III_a$) est récupéré de la phase aqueuse obtenue par l'addition d'un solvant immiscible au moyen de l'acidification de ladite phase aqueuse jusqu'à une valeur de pH comprise entre 3,5 et 4,5 afin de précipiter tout l'acide ($III_a$) et de le retirer par filtration.

# INTERNATIONAL SEARCH REPORT

International Application No PCT/ES 91/00049

**I. CLASSIFICATION OF SUBJECT MATTER** (if several classification symbols apply, indicate all) [6]

According to International Patent Classification (IPC) or to both National Classification and IPC

Int. Cl.[5] C 07 C 201/16, 201/12, 205/57, 205/58, 205/59 , 205/60

**II. FIELDS SEARCHED**

Minimum Documentation Searched [7]

| Classification System | Classification Symbols |
|---|---|
| Int. Cl.[5] | C 07 C 201/00, 205/00 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | US, A, 4288615 (J.H. BRIGHT) 8 September 1981, see claims<br>--- | 11-24 |
| A | Methoden der Organischen Chemie, vol. IV/1a, 1981 Georg Thieme publisher, Stuttgart (DE) see pages 559-562<br>--- | 1-24 |
| A | The Journal of the Chemical Society, 1950, London, GB; W. Davey et al.: "The preparation of the mononitrobenzaldehydes", see pages 204-208, specially page 208 (cited in the application)<br>-------------------- | 1-10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 12 November 1991 (12.11.91) | 20 December 1991 (20.12.91) |
| **International Searching Authority** | **Signature of Authorized Officer** |
| European Patent Office | |